# EUROPEAN PATENT SPECIFICATION

(11) **EP 4 392 098 B1**
(45) Date of publication and mention of the grant of the patent: **14.10.2026**
(21) Application number: 22761562.2
(22) Date of filing: 10.08.2022
(51) Int. Cl.: A61M 5/24, A61M 5/28, A61M 5/31, A61M 5/34, A61J 1/06

(54) **MEDICAMENT DELIVERY DEVICE ASSEMBLY KIT AND METHOD OF ASSEMBLY**
MEDIKAMENTENABGABEVORRICHTUNGSMONTAGESATZ UND VERFAHREN ZUR MONTAGE
KIT D'ASSEMBLAGE DE DISPOSITIF D'ADMINISTRATION DE MÉDICAMENTS ET PROCÉDÉ D'ASSEMBLAGE

(30) Priority: 23.08.2021 EP 21192670
(43) Date of publication of application: 03.07.2024
(73) Proprietor: SHL Medical AG, 6302 Zug (CH)
(72) Inventor: SÄLL, Daniel, 131 28 Nacka Strand (SE)
(86) International application number: PCT/EP2022/072478
(87) International publication number: WO 2023/025592

(56) References cited:
- DE-B1- 2 056 688
- US-A1- 2011 270 220
- US-A1- 2020 276 082
- US-A1- 2020 297 580

## Description

### TECHNICAL FIELD

The invention concerns medicament delivery devices.

### BACKGROUND

Some medicament delivery devices need to be assembled prior to use by a medical professional or an end user. Exemplary drug delivery devices with pre-filled flexible containers are known from US 2020/297580 and US 2011/270220.

However, this process can be complicated and require a number of steps. It has been appreciated that there is potential for changing medicament delivery device design to provide simpler device assembly.

### SUMMARY

The invention is defined by the appended claims, to which reference should now be made.

In the present disclosure, when the term "distal direction" is used, this refers to the direction pointing away from the dose delivery site during use of the medicament delivery device. When the term "distal part/end" is used, this refers to the part/end of the delivery device, or the parts/ends of the members thereof, which under use of the medicament delivery device is/are located furthest away from the dose delivery site. Correspondingly, when the term "proximal direction" is used, this refers to the direction pointing towards the dose delivery site during use of the medicament delivery device. When the term "proximal part/end" is used, this refers to the part/end of the delivery device, or the parts/ends of the members thereof, which under use of the medicament delivery device is/are located closest to the dose delivery site.

Further, the terms "longitudinal", "longitudinally", "axially" and "axial" refer to a direction extending from the proximal end to the distal end and along the device or components thereof, typically in the direction of the longest extension of the device and/or component.

Similarly, the terms "transverse", "transversal" and "transversally" refer to a direction generally perpendicular to the longitudinal direction.

A first aspect concerns a medicament delivery device assembly kit comprising a primary package and a housing, wherein the housing and the primary package each extend along an axis in an axial direction from a distal end to a proximal end, wherein the housing defines a cavity, the cavity extending in the axial direction from the proximal end of the housing, the cavity being configured to receive the distal end of the primary package. The primary package comprises a medicament container containing a medicament and a medicament delivery member container attached to the proximal end of the medicament container, the medicament delivery member container containing a medicament delivery member, wherein the medicament delivery member is spaced apart in the axial direction from the medicament, and wherein the medicament delivery member container is moveable in the axial direction relative to the medicament container. The medicament container comprises an attachment portion and the housing comprises a corresponding attachment mechanism configured to attach to the attachment portion of the medicament container so as to hold the primary package in place in the cavity of the housing. The force required to attach the attachment portion of the medicament container to the attachment mechanism of the housing is larger than the force required for the medicament delivery member container to move towards the distal end relative to the medicament so that the medicament delivery member moves into contact with the medicament. This can help ensure that the medicament delivery member is correctly inserted into the medicament container when the housing and the primary package are attached together.

The attachment portion of the medicament container is a protrusion at the distal end of the medicament container. The protrusion extends in the axial direction. Optionally, the attachment mechanism of the housing is one or more flexible fingers, and the force required for the protrusion of the primary package to flex the flexible fingers so that the protrusion extends between the flexible fingers is higher than the force required for the medicament delivery member container to move towards the distal end relative to the medicament so that the medicament delivery member moves into contact with the medicament.

The attachment portion of the primary package is a protrusion at the distal end of the medicament container extending in the axial direction.

Optionally, the attachment mechanism of the housing is a protrusion extending perpendicular to the axis.

Optionally, the protrusion comprises a pointed end that points towards the axis. This can allow the protrusion of the housing to penetrate the protrusion of the primary package.

Optionally, the attachment portion of the primary package and the attachment mechanism of the housing are the two halves of a snap-fit connection, and the force required to connect the two halves of the snap-fit connection together is higher than the force required for the medicament delivery member container to move towards the distal end relative to the medicament so that the medicament delivery member moves into contact with the medicament.

The primary package is a flexible primary package, and wherein the housing comprises a squeezing mechanism to expel the medicament from the medicament container. Optionally, the squeezing mechanism comprises a housing and a button moveable relative to the housing in the axial direction, wherein the button comprises an arm with a recess or cut-out. Optionally, the housing comprises two flexible arms, each arm comprising a protrusion extending into the recess or cut-out. Optionally, the housing comprises an outer housing and an inner housing attached inside the outer housing, and wherein the button is attached to the outer housing. Optionally, the recess or cut-out is wider at the proximal end than at the distal end. Optionally, the protrusions are arranged in the proximal end of the recess or cut-out. Optionally, the inner housing comprises a cut-out, and the flexible fingers are attached to the inner housing adjacent to the cut-out.

Optionally, the squeezing mechanism comprises a rack, a corresponding cog that rotates when the rack is moved past the cog in the axial direction, a cylinder attached to the cog and a tube inside the cylinder. Rotating the cylinder inside the tube results in the tube moving towards the axis and thereby providing a squeezing action due to a helical structure with which the cylinder and the tube are connected to one another. Optionally, and more generally, the tube is rotationally fixed relative to one of the cog and the tubular portion and rotates relative to the other of the cog and the tubular portion, thereby providing a squeezing action.

Optionally, the medicament delivery member container is a needle cover. Optionally, the medicament delivery member is a needle. Optionally, when the primary package is attached to the housing, the axis of the primary package and the axis of the housing are the same axis. Optionally, the primary package is a blow-fill-seal primary package. Optionally, the medicament delivery device assembly kit is an injection device assembly kit for an injection device such as a pen injector or an autoinjector.

Optionally, the medicament delivery device is an injection device such as a pen injector or an autoinjector.

A further aspect concerns a method of assembling a medicament delivery device, comprising the steps of: providing a medicament delivery device assembly kit as described above; and, whilst gripping the primary package by the medicament delivery member container and gripping the housing, pushing the primary package into the housing so that the primary package is attached to the housing and so that the medicament delivery member container is pushed in the distal direction relative to the rest of the primary package, resulting in the medicament delivery member coming into contact with the medicament.

Generally, all terms used in the claims are to be interpreted according to their ordinary meaning in the technical field, unless explicitly defined otherwise herein. All references to a/an/the element, apparatus, member, component, means, etc. are to be interpreted openly as referring to at least one instance of the element, apparatus, member component, means, etc., unless explicitly stated otherwise.

### BRIEF DESCRIPTION OF THE DRAWINGS

Embodiments of the present disclosure will now be described by way of example only and with reference to the accompanying drawings, in which:
Figure 1 shows a perspective view of an example medicament delivery device.
Figure 2 shows a perspective view of the housing of Figure 1.
Figure 3 shows a perspective view of the primary package of Figure 1.
Figures 4, 5 and 6 show perspective views of components of the housing of Figure 1.
Figure 7 shows a perspective cross-section view of part of the medicament delivery device of Figure 1.
Figure 8 shows a perspective view of part of the housing of Figure 1.
Figure 9 shows a perspective view of the outer housing of the housing of Figure 1.
Figure 10A shows a cross-section view of part of the medicament delivery device of Figure 1 before the needle cover is moved in the distal direction relative to the medicament container.
Figure 10B shows a cross-section view of part of the medicament delivery device of Figure 1 after the needle cover is moved in the distal direction relative to the medicament container and before the needle cover is removed.
Figure 11 shows a cross-section view of part of the medicament delivery device of Figure 1 after the needle cover is removed.
Figure 12 shows a perspective view of the medicament delivery device of Figure 1 with the needle cover removed.
Figures 13 and 14 show a side view of alternative medicament delivery device attachment and squeezing mechanisms.
Figure 15 shows a side view of the tube 134 of Figures 13 and 14.
Figure 16 shows a perspective and partially see-through view of another attachment mechanism.

In the Figures, a number of features and components are shown in dashed lines to show details of features and components behind them that would otherwise not be visible. The use of dashed lines is merely to aid understanding and is not intended to imply that the features and components shown with dashed lines are somehow different from the features and components shown with solid lines.

### DETAILED DESCRIPTION

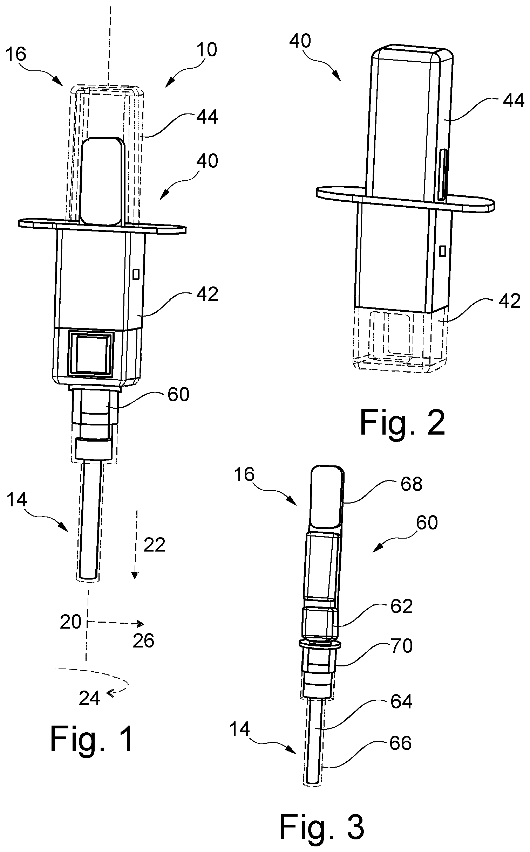

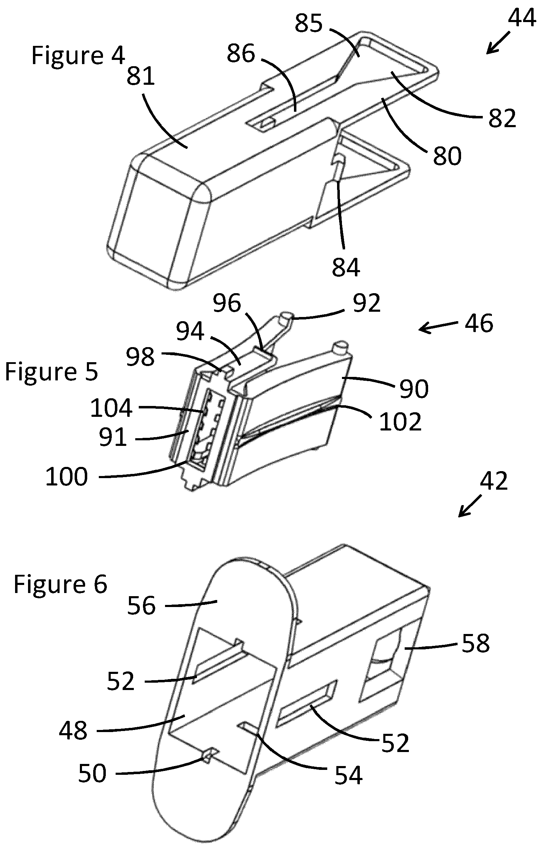

Figure 1 shows an example of a medicament delivery device 10, in this case a pen injector. The medicament delivery device 10 extends from a proximal end 14 to a distal end 16 in an axial direction 22 (longitudinal direction) along a longitudinal axis 20. The medicament delivery device 10 is made from a kit comprising two parts, namely a housing 40 and a primary package 60. As can be seen in more details in Figures 2 and 4 to 8, the housing 40 comprises an outer housing 42, a button 44 and an inner housing 46. As can be seen in more detail in Figure 3, the primary package 60 comprises a medicament container 62, a needle 64, a needle cover 66, a protrusion 68 and a cap 70.

The example housing 40 will now be described in more detail. The button 44 is arranged (partially) inside the outer housing 42 and the inner housing 46 is arranged inside the button 44. The button 44 is attached to the outer housing 42 by a snap-fit protrusion 84 of the button 44 and a corresponding slot 52 of the outer housing 42. The snap-fit protrusion 84 is arranged in the distal end of the slot (or recess) 52 (which allows the button 44 to move in the proximal direction relative to the outer housing 42 but not in the distal direction relative to the outer housing 42).

The button 44 comprises a body 81 and two arms 80 extending in the proximal direction from the proximal end of the body 81. The button 44 comprises two cut-outs 82. Each cut-out extends from a distal end in the body to a proximal end in one of the arms 80. As a result, each arm includes one cut-out. A distal portion 86 of each cut-out is rectangular, and a proximal portion of each cut-out is triangular. The proximal end of the distal portion 86 is joined to a distal corner of the triangular portion 85.

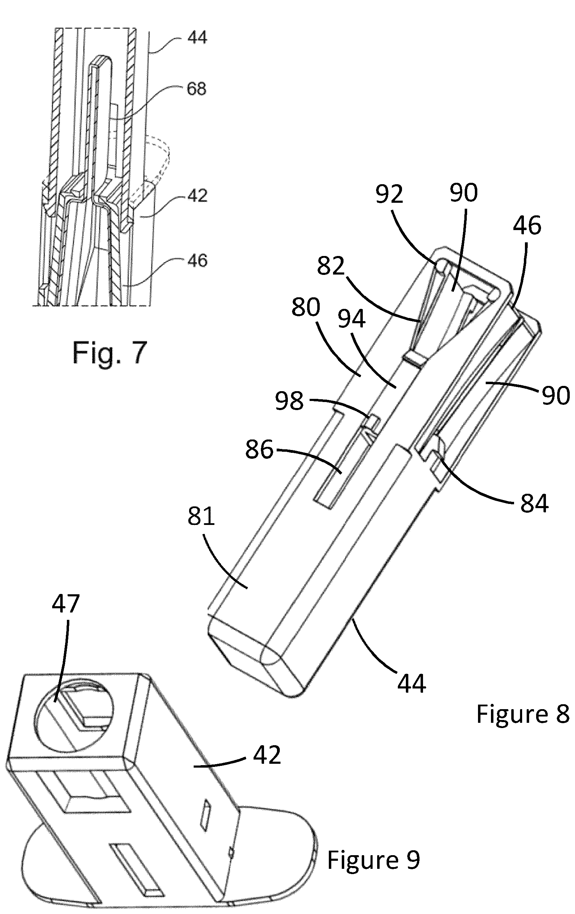

The inner housing 46 comprises a body 91 and two arms 90 extending in the proximal direction from the proximal end of the body 91. At the proximal end of each arm (though they could also be spaced apart from the proximal end of the arm), a pair of protrusions 92 extend perpendicular to the axis 20 (in Figure 8, for example, one protrusion of each pair of protrusions is visible). The arms 90 are flexible, and can flex towards each other; an optional longitudinally-extending rib 102 can be provided to increase the stiffness of the arm 90. The body 91 comprises a cut-out 100 (attachment mechanism), which is configured to receive an attachment portion of the primary package (which in this example is a distal portion of the primary package, namely the protrusion 68 of the primary package 60). A plurality of flexible teeth 104 extend from the body 91 into the cut-out 100; these teeth are provided to engage the attachment portion of the primary package. The flexibility of the teeth could be adjusted in different examples, for example by varying the thickness of the teeth based on the amount of force that is required to attach the attachment portion of the medicament container to the attachment portion of the housing. This could help ensure that, even for different primary packages, the force required to attach the attachment portion of the medicament container to the attachment portion of the housing is larger than the force required for the medicament delivery member container to move towards the distal end relative to the medicament so that the medicament delivery member moves into contact with the medicament.

The inner housing 46 also comprises a pair of second arms 94; these arms extend in the proximal direction from the body 91. A protrusion 96 is provided at the proximal end of each of the second arms 94; the protrusion extends in a plane perpendicular to the axis 20. The protrusion 96 engages a corresponding cut-out (or recess) 54 on the outer housing 42; the protrusion 96 and the recess 54 provide a snap-fit attachment between the outer housing 42 and the inner housing 46. The snap-fit attachment between the recess 54 and the protrusion 96 can stop the inner housing 46 moving in the distal direction relative to the outer housing 42; depending on the shape of the snap-fit attachment, this attachment could be sufficient to also stop the inner housing 46 moving in the proximal direction relative to the outer housing 42. In this particular example, an optional second protrusion 98 of the inner housing 46 is provided; the second protrusion 98 extends in a plane perpendicular to the axis 20 and extends from the distal end of the second arm 94. The second protrusion 98 engages a notch 50 of the outer housing 42 in this example.

The outer housing 42 is tubular and extends from a proximal opening 47 (see Figure 9) to a distal opening 48. In addition to the notch 50, the slot 52 and the recess 54 as described above, the outer housing 42 also comprises an optional pair of flanges 56 and optional viewing windows 58. The flanges 56 can help an end user to grip the medicament delivery device. The windows 58 can allow the medicament in the primary container to be visible to an end user.

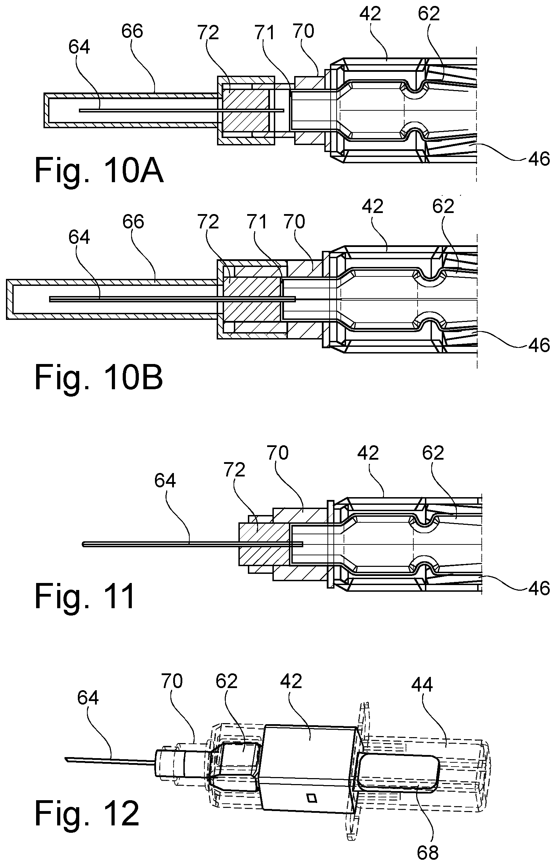

The primary package shown in this example (particularly in Figure 3) is an example of a BFS (blow-fill-seal) primary package. A medicament container 62 contains a medicament. The medicament container 62 comprises a protrusion 68 extending at the distal end of the medicament container 62, a cap 70 at the proximal end of the medicament container 62, and a seal 71 at the proximal end of the medicament container 62 (inside the cap 70). In this example, the seal is effectively the front wall of the medicament container 62. The primary package also comprises a needle container comprising a needle 64, a needle cover 66 and a needle support 72 (see Figure 10 in particular). The needle 64 is attached to the needle support 72, and the needle cover 66 is arranged around the needle 64. The needle container is attached to the medicament container 62; specifically in this example, the needle cover 66 and the needle support 72 are both attached to the cap 70 by a friction fit, although only one of these friction fit attachments is necessary, and other alternative (removable) attachments are also possible. There is preferably a gap in the axial direction between the needle support 72 and the seal 71; this can allow for manufacturing tolerances and make it harder for the needle to accidentally penetrate the seal. When this primary package is to be used, the needle cover 66 is pushed in the distal direction relative to the medicament container 62. This moves the needle 64 and the needle support 72 in the distal direction relative to the medicament container 62 along with the needle cover 66, and as a result the needle 64 (specifically the distal end of the needle) penetrates the seal 71 and comes into contact with the medicament in the medicament container 62 (see Figure 10B). This is one example design, and could be modified. For example, the cap 70 is optional. One example of this type of primary package is the Uniject^{®}, which is produced by Becton Dickinson.

To assemble a medicament delivery device as described above, the housing is initially assembled by inserting the inner housing 46 into the button 44 (this combination of components is shown in Figure 8). The protrusions 92 and the second arm 94 (or at least the protrusions 96, 98 of the second arm) protrude into the cut-outs 82. The combination of the inner housing 46 and the button 44 is then inserted into the outer housing 42 (in this case by inserting the combination of the inner housing 46 and the button 44 into the distal end of the outer housing 42), resulting in the housing 40 as shown in Figure 2.

The medicament delivery device could be stored and/or transported as a kit of two parts, namely the housing and the primary package, or the housing and the primary package could be attached together and then stored. It can be beneficial to only attach the housing and the primary package together shortly before use (for example, a few minutes, hours or days before use), as this can allow the needle to remain isolated from the medicament until shortly before use.

To attach the housing and the primary package together, the distal end of the primary package is inserted into the proximal opening 47 of the outer housing 42 (and therefore into the cavity defined by the housing) to complete assembly of the medicament delivery device. When the primary package is inserted into the housing, the primary package is held at the needle container. As a result, pushing the primary package in the distal direction relative to the housing results in a force in the distal direction on the medicament container from the needle container and a force in the proximal direction on the medicament container from the housing.

At the distal end of the primary package, the result as the primary package 60 moves in the distal direction relative to the housing 40 is that the protrusion 68 of the primary package 60 passes through the cut-out 100, thereby engaging the teeth 104 with the protrusion 68. The force required for the protrusion 68 to push the teeth aside and pass through the cut-out 100 is greater than the force required for the needle cover 66 of the needle container to be pushed in the distal direction relative to the medicament container 62 (thereby pushing the needle support 72 and the needle 64 in the distal direction relative to the medicament container 62 and resulting in the seal 71 being penetrated by the needle 64). The needle 64 therefore finishes in contact with the medicament in the medicament container 62.

To use the example device shown in Figure 1, the needle cover 66 is first removed. The needle 64 is then inserted into an injection site, and the button 44 is then pushed in the proximal direction relative to the outer housing 42, resulting in the primary package (and particularly the medicament container 62) being squeezed by the mechanism described above, resulting in expulsion of the medicament from the medicament container 62. In examples where the manual squeezing mechanism has been replaced by an automatic squeezing mechanism (for example a motor-driven squeezing mechanism), injection could be activated by flipping a switch on the housing or pushing a button on the housing, for example.

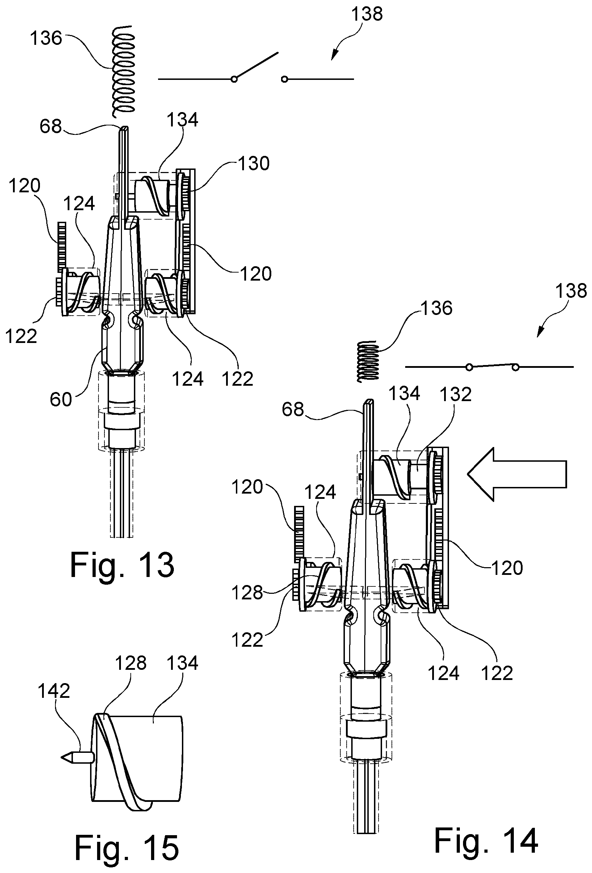

In general, the housing can be considered to comprise a squeezing mechanism (to squeeze medicament out of a medicament container), which is made up of elements of the outer housing 42, the button 44 and the inner housing 46. The squeezing mechanism can be changed - in the example in Figures 1 to 12, it comprises a cut-out 82 and a pair of protrusions 92 that are configured to transfer force in the longitudinal direction to force pushing towards the axis 20 when the button 44 is pushed in the proximal direction relative to the outer housing 42, but could be replaced by other squeezing mechanisms, such as the one described below with reference to Figures 13 and 14. In general, the squeezing mechanism is configured to transfer force in the longitudinal direction to force towards the axis.

Figures 13 to 15 show another example in which alternative solutions are shown for both the squeezing mechanism and for the attachment mechanism by which the primary package and the housing are attached together.

The squeezing mechanism described with reference to Figures 1 to 12 uses a cut-out 82 and a pair of protrusions 92 to transfer force in the longitudinal direction to force pushing towards the axis 20 when the button 44 is pushed in the proximal direction relative to the outer housing 42. The squeezing mechanism shown in Figures 13 and 14 instead provides this force transfer using a rack and pinion system to transfer force in the longitudinal direction to a rotational force. In short, movement of the racks 120 (which are attached to the button 44) in the proximal direction relative to the cogs 122 (which are attached to and rotatable relative to the outer housing 42) causes the cogs 122 to rotate. The cogs 122 are each attached to a cylinder (not shown, although they would be the same as or similar to the cylinder 132 described below). Each cylinder is inside a tube 124 and engages with the tube 124 by a spline (for example a rib on one of the tube and the cylinder and a recess on the other of the tube and the cylinder, with the rib typically extending towards the primary package). Each of the tubes 124 comprises a thread 128 on the outer surface of the tube. Each tube is arranged inside a corresponding tubular portion of the housing (not shown). The thread 128 engages a corresponding feature (for example a thread, a protrusion or a rib) on the tubular portion of the housing (alternatively, the thread 128 could be on the tubular portion of the housing rather than on the tube). As a result, the tubes 124 can rotate relative to the tubular portion of the housing. Therefore, the tubes 124 can move in a direction perpendicular to the longitudinal direction but are restricted from movement in other directions. As a result, when the cogs 122 (and therefore the threaded cylinders) are rotated, the tubes 124 are also rotated and are therefore pushed towards the longitudinal axis of the device (and therefore towards each other), squeezing the medicament container and therefore squeezing the medicament out of the medicament container. A more detailed description of the squeezing mechanism concept and of other alternative squeezing mechanisms that could be used instead of the squeezing mechanism described herein are provided in EP patent application no. EP21157206.0 (in particular the squeezing mechanisms described therein, including page 11 line 3 to page 24 line 12 of the description and Figures 1 to 10). The squeezing mechanism could be activated manually (in a pen injector) or driven, for example by a motor (in an autoinjector). The squeezing mechanism shown in Figures 13 and 14 could be replaced by other squeezing mechanisms, for example the one described above using an inner housing.

The attachment mechanism by which the primary package and the housing are attached together, as shown in Figures 13 to 15, comprises a rack and pinion system similar to that described as part of the squeezing mechanism above. A rack on the button 44 (not shown in Figures 13 to 15) of the housing engages with a cog 130. The cog 130 is attached to a cylinder 132. The cylinder is in a tube 134. A spline is provided between the cylinder 132 and the tube 134 (for example a rib on one of the tube and the cylinder and a recess on the other of the tube and the cylinder, with the rib typically extending towards the primary package). The tube 134 is therefore locked from rotation relative to the cylinder 132 but can move towards the longitudinal axis relative to the cylinder 132. The tube 134 is inside a tubular portion 144 of the housing. A thread 128 of the tube 134 engages a corresponding feature (for example a thread, a protrusion or a rib) on the tubular portion of the housing (alternatively, the thread 128 could be on the tubular portion of the housing rather than on the tube). As a result, the tube 134 is able to move in the radial direction 26 relative to the axis, as shown in Figure 13 (before radial movement) and Figure 14 (after radial movement). This engages the attachment mechanism (attachment portion) of the housing with the attachment mechanism (attachment portion) of the primary package - in this case by driving a protrusion 142 (see Figure 15) into the protrusion 68 of the primary package 60 (for example into a hole in the protrusion 68, into a recess in the protrusion 68, or by punching a hole into or through the protrusion 68).

The attachment mechanism is triggered as follows. When the primary package is inserted into the housing, a spring 136 in the housing is compressed by the primary package. A switch 138 (shown only in the abstract in Figures 13 and 14) is arranged at the distal end of the spring. The switch could be mechanical (flipping once a certain force is applied to it by the spring, and thereby physically rotating the cog 130 of the attachment mechanism). Alternatively, the squeezing mechanism could be electrical, for example with a pressure sensor at the distal end of the spring detecting the force transmitted by the compression of the spring and activating the squeezing mechanism, with the squeezing mechanism driven by an electric motor. Depending on the primary package, the force required to flip the switch can be varied; this could help ensure that, even for different primary packages, the force required to attach the attachment portion of the medicament container to the attachment portion of the housing is larger than the force required for the medicament delivery member container to move towards the distal end relative to the medicament so that the medicament delivery member moves into contact with the medicament.

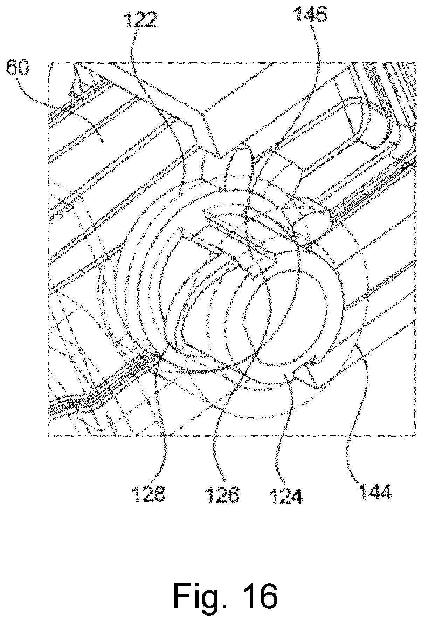

Figure 16 illustrates a further example of a similar squeezing mechanism to the ones described in Figures 13 to 15 - this approach could be used to replace some or all of the equivalent squeezing mechanisms and attachment mechanisms in Figures 13 to 15. In this example, the cog 122 is arranged at the end of the tube 124 closest to the longitudinal axis rather than at the end of the tube 124 furthest from the longitudinal axis, and as a result, a cylinder such as cylinder 132 (that is attached to the cog) is not necessary.

In this example, the cog 122 comprises an internal thread (not shown)(or other feature such as a protrusion) to engage a thread 128 of the tube 124. The tube 124 is rotationally locked relative to the tubular portion 144 by a spline (in this case a recess 126 in the tube 124 which engages a radially inwardly extending rib 146 of the tubular portion 144, although the recess could be on the tubular portion instead, with the rib on the tube). As a result, when the cog rotates, the tube is moved towards the longitudinal axis.

In general, in the squeezing mechanisms described above using a tube, the tube is rotationally fixed relative to one of the cog and the tubular portion and rotates relative to the other of the cog and the tubular portion. In Figures 13 to 15, the tube is rotationally fixed relative to the cog and rotates relative to the tubular portion. In Figure 16, the tube rotates relative to the cog and is rotationally fixed relative to the tubular portion.

The designs described herein could be used in various medicament delivery devices, and particularly in injectors such as pen injectors and autoinjectors. As mentioned above, a motor could be used to actuate the squeezing mechanism to provide an autoinjector rather than a pen injector. This would be possible for either of the main examples outlined above, but could be particularly suitable for the example shown in Figures 13 to 15.

Much of the structure within the devices described herein is symmetrical, for example with two arms 80, two cut-outs 82, two recesses 126 and so on. In general, a single arm 80, cut-out 82, recess 126 and so on could alternatively be provided, although it does tend to be beneficial to provide these features in symmetrical pairs, for example to help with load distribution and to help with effectively dispensing a medicament from the primary package.

An exemplary housing is described herein, but could be varied in structure. For example, the outer housing 42 could have a circular cross-section perpendicular to the longitudinal axis rather than a rectangular cross-section. The outer housing 42 described herein has openings 47, 48 at both its proximal end and its distal end, though the distal end opening would not be necessary in a motor-driven device as the button 44 would then be optional (an activation means could instead be provided elsewhere on the device) - as such, the housing could more generally be a housing that defines a cavity, the cavity extending in the axial direction from the proximal end of the housing (i.e. from the proximal opening 47). A see-through portion could be provided on the outer housing 42 instead of window 58. The button 44 and the inner housing 46 could also vary in shape. For example, the cut-out 82 could instead be a recess. The cut-out could also be a different shape, as long as the cut-out extends from a narrower distal end to a wider proximal end. The arms 80 of the button 44 could be flexible to help with assembly. The two protrusions of each pair of protrusions 92 extend in opposite directions from each arm, though this is not essential, and is (partly) dependent on the shape of the arm of the button 44. If the button 44 was cylindrical in shape, for example (rather than a cuboid as in the Figures), the cut-out (or recess) 82 in the button 44 could curve around the circumference of the button, and the protrusions would typically not extend in opposite directions but would each extend away from the longitudinal axis. The arm 90 of the inner housing 46 could just consist of the longitudinally-extending rib 102, with the protrusions 92 extending directly from the rib 102. Instead of being flexible, arm 90 of the inner housing 46 could instead be arranged to pivot so as to allow the protrusions 92 to move closer together. The second arm 94 of the inner housing 46 could extend in the distal direction rather than the proximal direction, and the second protrusion 98 could be provided on the body 91 rather than on the second arm 94. Other fixtures for holding the outer housing 42 and inner housing 46 in place relative to each other could be provided instead of the second arm 94, and more generally the second arm 94 is optional as the outer housing 42 and inner housing 46 can just be held in place in place relative to each other indirectly by the button 44. The outer housing 42 and inner housing 46 could be a single integral part (a housing) rather than two separate parts. The shape of the cut-out 100 (which is rectangular in the example above) could be varied as well - typically the shape is dependent on the shape of a distal portion of the primary package. The cut-out 100 (and the corresponding teeth 104) could be part of the outer housing 42 rather than of the inner housing 46.

Although the designs described above focus on a BFS primary package, the concepts described herein could also be used with other types of flexible primary package. For example, a cartridge with a distal flange could be used, where the attachment portion of the medicament container is the distal flange. In general, the concept could be used in devices with a needleless cartridge, where the needle is provided separately to the medicament container. The primary package could contain an RFID tag, which could be attached to the protrusion 68, for example.

The attachment concepts used herein could also be used with non-flexible primary packages. In general, the provision of a squeezing mechanism is therefore optional, and in particular would not be provided when a non-flexible primary package is used - an alternative powerpack or other medicament dispensing mechanism could be used instead, for example a solution using a plunger rod to expel the medicament from a cartridge.

A needle is described herein as an exemplary medicament delivery member, but other medicament delivery members such as a jet injector could be used instead. The needle described herein can therefore be generalised to a medicament delivery member, and the needle cover 66 can therefore also be generalised to a medicament delivery member cover.

Various alternatives are also available for the attachment portions 68, 100, 134 of the housing and of the medicament container. To help stop the protrusion from passing back through the teeth 104 and thereby falling out in the example described in Figures 1 to 12, a ledge or a protrusion extending perpendicular to the longitudinal axis could be provided. This ledge could be a proximal end of an RFID tag. A snap fit could be provided instead of the protrusion and teeth shown in Figures 1 to 12 or instead of the protrusion and cog, cylinder, and tube solution shown in Figures 13 to 15. The teeth 104 could be made of plastic or metal, for example.

Various other alternatives are also possible for the example shown in Figures 13 to 15. For example, a single attachment mechanism is shown in Figures 13 and 14, but a second attachment mechanism could be provided opposite the attachment mechanism; this could help balance the forces on the device and could help keep the primary package aligned with the longitudinal axis. As described above, the cylinder 132 and the tube 134 have corresponding screw threads (so a helical rib and a corresponding helical recess). Alternatively, as only one side needs a helical structure, the helical rib could be replaced by a protrusion. The protrusion would protrude into the helical recess. Provision of a protrusion rather than a helical rib could reduce friction. The same alternative is also possible for the tube 124 and the corresponding cylinder of the squeezing mechanism. As shown in Figure 15, the end of the protrusion 142 pointing towards the axis is preferably pointed (e.g. conical or a truncated cone) to minimise the force required to penetrate the protrusion 68 of the primary package.

## Claims

1. A medicament delivery device assembly kit comprising a primary package (60) and a housing (40),
wherein the housing (40) and the primary package (60) each extend along an axis in an axial direction from a distal end to a proximal end,
wherein the housing (40) defines a cavity, the cavity extending in the axial direction from the proximal end of the housing (40), the cavity being configured to receive the distal end of the primary package (60),
the primary package (60) comprising
a medicament container (62) containing a medicament and
a medicament delivery member container attached to the proximal end of the medicament container (62),
the medicament delivery member container containing a medicament delivery member (64),
wherein the medicament delivery member (64) is spaced apart in the axial direction from the medicament,
and wherein the medicament delivery member container is moveable in the axial direction relative to the medicament container (62),
wherein the medicament container (62) comprises an attachment portion (68) and the housing comprises a corresponding attachment mechanism (100, 134) configured to attach to the attachment portion (100) of the medicament container (62) so as to hold the primary package (60) in place in the cavity of the housing (40), and
wherein the attachment portion (68) and the attachment mechanism (100, 134)are further configured such that during assembly of the primary package (60) to the housing (40), the force required to attach the attachment portion (68) of the medicament container (62) to the attachment mechanism (100, 134) of the housing (40) so that the medicament delivery member (64) finishes in contact with the medicament in the medicament container (62) is larger than the force required for the medicament delivery member container to move towards the distal end relative to the medicament so that the medicament delivery member (64) moves into contact with the medicament,
wherein the attachment portion (68) of the medicament container (62) is a protrusion at the distal end of the medicament container (62) and wherein the protrusion extends in the axial direction,
and wherein the primary package is a flexible primary package, and wherein the housing comprises a squeezing mechanism to expel the medicament from the medicament container (62).

2. The medicament delivery device assembly kit of claim 1, wherein the attachment mechanism of the housing is one or more flexible fingers, and
wherein the force required for the protrusion of the primary package to flex the flexible fingers so that the protrusion extends between the flexible fingers is higher than the force required for the medicament delivery member container to move towards the distal end relative to the medicament so that the medicament delivery member moves into contact with the medicament.

3. The medicament delivery device assembly kit of claim 1, wherein the attachment mechanism of the housing is a protrusion (142) extending perpendicular to the axis.

4. The medicament delivery device assembly kit of claim 3, wherein the protrusion comprises a pointed end that points towards the axis.

5. The medicament delivery device assembly kit of claim 1, wherein the attachment portion of the primary package and the attachment mechanism of the housing are the two halves of a snap-fit connection, and the force required to connect the two halves of the snap-fit connection together is higher than the force required for the medicament delivery member container to move towards the distal end relative to the medicament so that the medicament delivery member moves into contact with the medicament.

6. The medicament delivery device assembly kit of any one of the preceding claims, wherein the squeezing mechanism comprises a housing and a button moveable relative to the housing in the axial direction, wherein the button comprises an arm with a recess or cut-out, and
wherein the housing comprises two flexible arms, each arm comprising a protrusion extending into the recess or cut-out.

7. The medicament delivery device assembly kit of claim 6, wherein the housing comprises an outer housing and an inner housing attached inside the outer housing, and wherein the button is attached to the outer housing.

8. The medicament delivery device assembly kit of claim 6 or 7, where the recess or cut-out is wider at the proximal end than at the distal end, and the protrusions are arranged in the proximal end of the recess or cut-out.

9. The medicament delivery device assembly kit of a combination of claim 2 and either of claims 7 and 8, wherein the inner housing comprises a cut-out, and wherein the flexible fingers are attached to the inner housing adjacent to the cut-out.

10. The medicament delivery device assembly kit of any one of the preceding claims, wherein the squeezing mechanism comprises a rack, a corresponding cog that rotates when the rack is moved past the cog in the axial direction, a cylinder attached to the cog and a tube inside the cylinder, wherein rotating the cylinder inside the tube results in the tube moving towards the axis and thereby providing a squeezing action due to a helical structure with which the cylinder and the tube are connected to one another.

11. The medicament delivery device assembly kit of any previous claim, wherein the medicament delivery member container is a needle cover and the medicament delivery member is a needle.

12. The medicament delivery device assembly kit of any previous claim, wherein the primary package is a blow-fill-seal primary package.

13. A medicament delivery device comprising the primary package (60) and the housing (40) of any previous claim.

14. A method of assembling a medicament delivery device, comprising the steps of:
providing a medicament delivery device assembly kit according to any of claims 1 to 12; and,
whilst gripping the primary package by the medicament delivery member container and gripping the housing, pushing the primary package into the housing so that the primary package is attached to the housing and so that the medicament delivery member container is pushed in the distal direction relative to the rest of the primary package, resulting in the medicament delivery member coming into contact with the medicament.

## Patentansprüche

1. Montagesatz für eine Arzneimittelabgabevorrichtung, umfassend eine Primärverpackung (60) und ein Gehäuse (40),
wobei sich das Gehäuse (40) und die Primärverpackung (60) jeweils entlang einer Achse in einer axialen Richtung von einem distalen Ende zu einem proximalen Ende erstrecken,
wobei das Gehäuse (40) einen Hohlraum definiert, wobei sich der Hohlraum in der axialen Richtung von dem proximalen Ende des Gehäuses (40) erstreckt, wobei der Hohlraum konfiguriert ist, um das distale Ende der Primärverpackung (60) aufzunehmen,
die Primärverpackung (60), umfassend
einen Arzneimittelbehälter (62), der ein Arzneimittel enthält, und
einen Arzneimittelabgabeelementbehälter, der an dem proximalen Ende des Arzneimittelbehälters (62) befestig
der Arzneimittelabgabeelementbehälter, der ein Arzneimittelabgabeelement (64) enthält,
wobei das Arzneimittelabgabeelement (64) in der axialen Richtung von dem Arzneimittel beabstandet ist,
und wobei der Arzneimittelabgabeelementbehälter in der axialen Richtung relativ zu dem Arzneimittelbehälter (62) bewegbar ist,
wobei der Arzneimittelbehälter (62) einen Befestigungsabschnitt (68) umfasst und das Gehäuse einen entsprechenden Befestigungsmechanismus (100, 134) umfasst, der konfiguriert ist, um an dem Befestigungsabschnitt (100) des Arzneimittelbehälters (62) befestigt zu werden, um die Primärverpackung (60) in dem Hohlraum des Gehäuses (40) an Ort und Stelle zu halten, und
wobei der Befestigungsabschnitt (68) und der Befestigungsmechanismus (100, 134) ferner derart konfiguriert sind, dass während einer Montage der Primärverpackung (60) an dem Gehäuse (40) die Kraft, die erforderlich ist, um den Befestigungsabschnitt (68) des Arzneimittelbehälters (62) an dem Befestigungsmechanismus (100, 134) des Gehäuses (40) zu befestigen, sodass das Arzneimittelabgabeelement (64) abschließend in Kontakt mit dem Arzneimittel in dem Arzneimittelbehälter (62) steht, größer als die Kraft ist, die erforderlich ist, damit sich der Arzneimittelabgabeelementbehälter relativ zu dem Arzneimittel zu dem distalen Ende hin bewegt, sodass sich das Arzneimittelabgabeelement (64) in Kontakt mit dem Arzneimittel bewegt,
wobei der Befestigungsabschnitt (68) des Arzneimittelbehälters (62) ein Vorsprung an dem distalen Ende des Arzneimittelbehälters (62) ist und wobei sich der Vorsprung in der axialen Richtung erstreckt,
und wobei die Primärverpackung eine flexible Primärverpackung ist, und wobei das Gehäuse einen Quetschmechanismus umfasst, um das Arzneimittel aus dem Arzneimittelbehälter (62) auszustoßen.

2. Montagesatz für die Arzneimittelabgabevorrichtung nach Anspruch 1, wobei der Befestigungsmechanismus des Gehäuses ein oder mehrere flexible Finger ist, und
wobei die Kraft, die erforderlich ist, damit der Vorsprung der Primärverpackung die flexiblen Finger biegt, sodass sich der Vorsprung zwischen die flexiblen Finger erstreckt, höher als die Kraft ist, die erforderlich ist, damit sich der Arzneimittelabgabeelementbehälter relativ zu dem Arzneimittel zu dem distalen End hin bewegt, sodass sich das Arzneimittelabgabeelement in Kontakt mit dem Arzneimittel bewegt.

3. Montagesatz für die Arzneimittelabgabevorrichtung nach Anspruch 1, wobei der Befestigungsmechanismus des Gehäuses ein Vorsprung (142) ist, der sich senkrecht zu der Achse erstreckt.

4. Montagesatz für die Arzneimittelabgabevorrichtung nach Anspruch 3, wobei der Vorsprung ein spitzes Ende umfasst, das zu der Achse hin zeigt.

5. Montagesatz für die Arzneimittelabgabevorrichtung nach Anspruch 1, wobei der Befestigungsabschnitt der Primärverpackung und der Befestigungsmechanismus des Gehäuses die zwei Hälften einer Schnappverbindung sind, und die Kraft, die erforderlich ist, um die zwei Hälften der Schnappverbindung miteinander zu verbinden, höher als die Kraft ist, die erforderlich ist, damit sich der Arzneimittelabgabeelementbehälter relativ zu dem Arzneimittel zu dem distalen Ende hin bewegt, sodass sich das Arzneimittelabgabeelement in Kontakt mit dem Arzneimittel bewegt.

6. Montagesatz für die Arzneimittelabgabevorrichtung nach einem der vorstehenden Ansprüche, wobei der Quetschmechanismus ein Gehäuse und einen Knopf umfasst, der relativ zu dem Gehäuse in der axialen Richtung bewegbar ist, wobei der Knopf einen Arm mit einer Aussparung oder einem Ausschnitt umfasst, und
wobei das Gehäuse zwei flexible Arme umfasst, jeder Arm umfassend einen Vorsprung, der sich in die Aussparung oder den Ausschnitt erstreckt.

7. Montagesatz für die Arzneimittelabgabevorrichtung nach Anspruch 6, wobei das Gehäuse ein Außengehäuse und ein Innengehäuse umfasst, das im Inneren des Außengehäuses befestigt ist, und wobei der Knopf an dem Außengehäuse befestigt ist.

8. Montagesatz für die Arzneimittelabgabevorrichtung nach Anspruch 6 oder 7, wobei die Aussparung oder der Ausschnitt an dem proximalen Ende breiter als an dem distalen Ende ist und die Vorsprünge in dem proximalen Ende der Aussparung oder des Ausschnitts angeordnet sind.

9. Montagesatz für die Arzneimittelabgabevorrichtung einer Kombination nach Anspruch 2 und einem der Ansprüche 7 und 8, wobei das Innengehäuse einen Ausschnitt umfasst, und wobei die flexiblen Finger an dem Innengehäuse angrenzend an den Ausschnitt befestigt sind.

10. Montagesatz für die Arzneimittelabgabevorrichtung nach einem der vorstehenden Ansprüche, wobei der Quetschmechanismus eine Zahnstange, ein entsprechendes Zahnrad, das sich dreht, wenn die Zahnstange in der axialen Richtung an dem Zahnrad vorbeibewegt wird, einen Zylinder, der an dem Zahnrad befestigte ist, und ein Rohr im Inneren des Zylinders umfasst, wobei das Drehen des Zylinders im Inneren des Rohrs dazu führt, dass sich das Rohr zu der Achse hin bewegt und dadurch aufgrund einer schraubenförmigen Struktur, mit der der Zylinder und das Rohr miteinander verbunden sind, eine Quetschwirkung bereitstellt.

11. Montagesatz für die Arzneimittelabgabevorrichtung nach einem vorstehenden Anspruch, wobei der Arzneimittelabgabeelementbehälter eine Nadelabdeckung ist und das Arzneimittelabgabeelement eine Nadel ist.

12. Montagesatz für die Arzneimittelabgabevorrichtung nach einem vorstehenden Anspruch, wobei die Primärverpackung eine Blasform-, Füll- und Verschließprimärverpackung ist.

13. Arzneimittelabgabevorrichtung, umfassend die Primärverpackung (60) und das Gehäuse (40) nach einem vorstehenden Anspruch.

14. Verfahren zum Montieren der Arzneimittelabgabevorrichtung, umfassend die Schritte:
Bereitstellen eines Montagesatzes für die Arzneimittelabgabevorrichtung nach einem der Ansprüche 1 bis 12; und,
während eines Greifens der Primärverpackung durch den Arzneimittelabgabeelementbehälter und des Greifens des Gehäuses, Schieben der Primärverpackung in das Gehäuse, sodass die Primärverpackung an dem Gehäuse befestigt wird und sodass der Arzneimittelabgabeelementbehälter in die distale Richtung relativ zu dem Rest der Primärverpackung geschoben wird, was dazu führt, dass das Arzneimittelabgabeelement mit dem Arzneimittel in Kontakt kommt.

## Revendications

1. Kit d'assemblage de dispositif d'administration de médicament comprenant un emballage primaire (60) et un boîtier (40),
dans lequel le boîtier (40) et l'emballage primaire (60) s'étendent chacun le long d'un axe dans une direction axiale depuis une extrémité distale à une extrémité proximale,
dans lequel le boîtier (40) définit une cavité, la cavité s'étendant dans la direction axiale depuis l'extrémité proximale du boîtier (40), la cavité étant conçue pour recevoir l'extrémité distale de l'emballage primaire (60),
l'emballage primaire (60) comprenant
un récipient à médicament (62) contenant un médicament et
un récipient à élément d'administration de médicament fixé à l'extrémité proximale du récipient à médicament (62),
le récipient à élément d'administration de médicament contenant un élément d'administration de médicament (64),
dans lequel l'élément d'administration de médicament (64) est écarté dans la direction axiale du médicament
et dans lequel le récipient à élément d'administration de médicament est mobile dans la direction axiale par rapport au récipient à médicament (62),
dans lequel le récipient à médicament (62) comprend une partie de fixation (68) et le boîtier comprend un mécanisme de fixation (100, 134) correspondant conçu pour se fixer à la partie de fixation (100) du récipient à médicament (62) de manière à maintenir l'emballage primaire (60) en place dans la cavité du boîtier (40) et
dans lequel la partie de fixation (68) et le mécanisme de fixation (100, 134) sont en outre conçus de telle sorte que, pendant l'assemblage de l'emballage primaire (60) au boîtier (40), la force requise pour la fixation de la partie de fixation (68) du récipient à médicament (62) au mécanisme de fixation (100, 134) du boîtier (40) de sorte que l'élément d'administration de médicament (64) finit en contact avec le médicament dans le récipient à médicament (62) est plus grande que la force requise pour le déplacement du récipient à élément d'administration de médicament vers l'extrémité distale par rapport au médicament de sorte que l'élément d'administration de médicament (64) entre en contact avec le médicament,
dans lequel la partie de fixation (68) du récipient à médicament (62) est une saillie au niveau de l'extrémité distale du récipient à médicament (62) et dans lequel la saillie s'étend dans la direction axiale
et dans lequel l'emballage primaire est un emballage primaire souple et dans lequel le boîtier comprend un mécanisme de serrage destiné à expulser le médicament à partir du récipient à médicament (62).

2. Kit d'assemblage de dispositif d'administration de médicament selon la revendication 1, dans lequel le mécanisme de fixation du boîtier représente un ou plusieurs doigts flexibles et
dans lequel la force requise pour le fléchissement des doigts flexibles par la saillie de l'emballage primaire de sorte que la saillie s'étend entre les doigts flexibles est plus élevée que la force requise pour le déplacement du récipient à élément d'administration de médicament vers l'extrémité distale par rapport au médicament de sorte que l'élément d'administration de médicament entre en contact avec le médicament.

3. Kit d'assemblage de dispositif d'administration de médicament selon la revendication 1, dans lequel le mécanisme de fixation du boîtier est une saillie (142) s'étendant perpendiculairement à l'axe.

4. Kit d'assemblage de dispositif d'administration de médicament selon la revendication 3, dans lequel la saillie comprend une extrémité pointue qui pointe vers l'axe.

5. Kit d'assemblage de dispositif d'administration de médicament selon la revendication 1, dans lequel la partie de fixation de l'emballage primaire et le mécanisme de fixation du boîtier sont les deux moitiés d'un raccord par encliquetage et la force requise pour le raccordement des deux moitiés du raccord par encliquetage ensemble est plus élevée que la force requise pour le déplacement du récipient à élément d'administration de médicament vers l'extrémité distale par rapport au médicament de sorte que l'élément d'administration de médicament entre en contact avec le médicament.

6. Kit d'assemblage de dispositif d'administration de médicament selon l'une quelconque des revendications précédentes, dans lequel le mécanisme de serrage comprend un boîtier et un bouton mobile par rapport au boîtier dans la direction axiale, dans lequel le bouton comprend un bras comportant un évidement ou une découpe et
dans lequel le boîtier comprend deux bras flexibles, chaque bras comprenant une saillie s'étendant dans l'évidement ou la découpe.

7. Kit d'assemblage de dispositif d'administration de médicament selon la revendication 6, dans lequel le boîtier comprend un boîtier externe et un boîtier interne fixé à l'intérieur du boîtier externe et dans lequel le bouton est fixé au boîtier externe.

8. Kit d'assemblage de dispositif d'administration de médicament selon la revendication 6 ou 7, où l'évidement ou la découpe est plus large au niveau de l'extrémité proximale qu'au niveau de l'extrémité distale et les saillies sont agencées dans l'extrémité proximale de l'évidement ou de la découpe.

9. Kit d'assemblage de dispositif d'administration de médicament selon une combinaison de la revendication 2 et de l'une des revendications 7 et 8, dans lequel le boîtier interne comprend une découpe et dans lequel les doigts flexibles sont fixés au boîtier interne de manière adjacente à la découpe.

10. Kit d'assemblage de dispositif d'administration de médicament selon l'une quelconque des revendications précédentes, dans lequel le mécanisme de serrage comprend une crémaillère, un pignon correspondant qui tourne lorsque la crémaillère est déplacée au-delà du pignon dans la direction axiale, un cylindre fixé au pignon et un tube à l'intérieur du cylindre, dans lequel la rotation du cylindre à l'intérieur du tube entraîne le déplacement du tube vers l'axe et fournit ainsi une action de serrage due à une structure hélicoïdale par laquelle le cylindre et le tube sont reliés l'un à l'autre.

11. Kit d'assemblage de dispositif d'administration de médicament selon l'une quelconque revendication précédente, dans lequel le récipient à élément d'administration de médicament est une protection d'aiguille et l'élément d'administration de médicament est une aiguille.

12. Kit d'assemblage de dispositif d'administration de médicament selon l'une quelconque revendication précédente, dans lequel l'emballage primaire est un emballage primaire obtenu par soufflage-remplissage-scellage.

13. Dispositif d'administration de médicament comprenant l'emballage primaire (60) et le boîtier (40) selon l'une quelconque revendication précédente.

14. Procédé d'assemblage d'un dispositif d'administration de médicament, comprenant les étapes consistant à :
fournir un kit d'assemblage de dispositif d'administration de médicament selon l'une quelconque des revendications 1 à 12 ; et,
lors de la préhension de l'emballage primaire par le récipient à élément d'administration de médicament et de la saisie du boîtier, la poussée de l'emballage primaire dans le boîtier de sorte que l'emballage primaire est fixé au boîtier et de sorte que le récipient à élément d'administration de médicament est poussé dans la direction distale par rapport au reste de l'emballage primaire, ce qui entraîne que l'élément d'administration de médicament entre en contact avec le médicament.
